# EUROPEAN PATENT APPLICATION

(11) **EP 4 364 714 A1**
(43) Date of publication of application: **08.05.2024**
(21) Application number: 23207293.4
(22) Date of filing: 01.11.2023
(51) Int. Cl.: A61H 21/00, A61H 1/02

(54) **RECTO-ANAL SEQUENCED EXPANDER**

(30) Priority: 03.11.2022 US 202263382132 P; 07.08.2023 US 202318366125
(71) Applicant: Backside Products LLC, Blaine, MN 55449 (US)
(72) Inventor: JOHNSTON, Evan S., Blaine (US); LAVENDER, Steven J., New York (US)
(74) Representative: Abel & Imray LLP

(57) **Abstract**

An apparatus of an elongated cylindrical shape (1, 2), which is inserted into the anal canal and expands progressively in segments, beginning with the distal end (3, 8) positioned in the rectal chamber, and finishing at or near the anal verge at the proximal end (4, 9). Expansion is gradually increased by the user as directed, and then decreased to its original dimensions, with a purpose of conditioning the anus for insertion of desired objects.

## Description

### Cross Reference to Related Applications

This application is a nonprovisional patent application which claims the benefit of United States Provisional Application Serial Number 63/382,132, filed on November 3, 2022, entitled "Recto-anal Sequenced Expander," the contents of which are incorporated herein in their entirety by reference thereto.

### Field of invention

The present invention relates generally to the art of anal dilation and more particularly to a device and method for sequentially and progressively expanding the anal canal of a person from the rectum outward toward the anal verge, thus helping to stretch and relax the surrounding muscles and tissues. This method harnesses the Rectoanal Inhibitory Reflex, the spinal reflex responsible for relaxing the internal anal sphincter and inhibiting reflexive anal sphincter closure. By utilizing this naturally occurring spinal reflex we can bypass the opposing body reflex, the Anal Wink Reflex, which is designed to close the anal sphincter against objects entering the anus from the outside. This allows one to more effectively and comfortably dilate the anal sphincter. This anal sphincter relaxation and dilation method and device is intended to be used as a preconditioning measure for many conditions that impact function and pleasure including, but not limited to, receptive anal intercourse, scar conditions and fibrosis of skin, anal spasm, anal stenosis and incomplete defecation.

### Background

The statements in this section merely provide background information related to the present disclosure and may not constitute prior art.

People who anticipate engaging in receptive anal intercourse, or the use of an anal plug or stimulator, may elect to precondition their anal canal, by stretching the muscles and tissues, to more comfortably accommodate the insertion of a penis or object, such as a dildo or plug. However, causing the anal canal to be expanded, by inserting an object or a penis, without first allowing the internal anal sphincter to achieve a relaxed state, may result in discomfort and/or physical tissue damage, such as a fissure or abrasion. Current therapies for medical conditions such as anal spasm, scaring and fibrosis and some defecation disorders rely on inserting incrementally larger plugs into the anus. The process can be painful and time consuming. Indeed, most currently available devices anatomically mimic a penis, or are either of constant diameter, or of gradually increasing diameter, such as conical in shape with the narrowest portion being inserted first. Conventional devices of this nature tend to force the anus to open from the exterior in an unnatural manner, thus increasing the potential for physical damage to the anal canal.

When the nerves within the rectal walls sense pressure, such as from the accumulation of feces or the presence of an object having a dimension such that it exerts some force upon the rectal walls, an autonomic reflex will naturally cause the internal anal sphincter muscles to relax. This action more easily allows the external, voluntary, anal sphincter to be relaxed prior to an object or penis being inserted. During sexual anal intercourse, this may provide greater satisfaction for both parties and reduce instances of discomfort or possible physical damage to the anal canal.

Therefore, it is evident that there is a distinct need and desire for an improved recto-anal expansion device which is capable of stimulating, relaxing and preconditioning the muscles and tissues surrounding the anal canal prior to engagement in receptive anal intercourse or inserting other devices. To this end, it is determined that the use of the natural recto-anal inhibitory reflex (RAIR), an involuntary neurological relaxation of the internal anal sphincter in response to distention of the rectum, may be beneficial to cause relaxation of the anal sphincter. An apparatus which simulates relaxation and anal distension as the initial action, and then expands the anorectal canal from the rectum outward toward the anus, utilizes the normal and natural physical functions to relax the canal, as if expelling a stool (feces). Consequently, further expansion using such an apparatus to a desired dimension is, therefore, likely to be more comfortable and less prone to cause physical damage to the anorectal tissues. It is with these objectives in mind, and more, that we have developed our improved recto-anal sequenced expander, as will be described in more detail below.

### Summary

In furtherance of the foregoing objectives, the present invention is comprised generally of an elongated cylindrically shaped recto-anal expander or dilator assembly having a relatively rigid inner tubular body member encased within or surrounded in sealed relation by an outer relatively flexible and expandable cover or sheath. The inner tubular body of the dilator is closed at its distal end and open at its proximal end to allow for the introduction of an expansion fluid therewithin. The sidewalls of the inner body extending between opposite ends thereof are perforated to allow passage of the expansion fluid from the inner tubular body to the outer expandable cover or sheath. As used herein and throughout the appended claims to describe the dilator, the term "distal" shall mean the end portion of the dilator which first enters the anus and is positioned within the rectum of the anal cavity, and the term "proximal" shall mean the opposite end portion of the dilator which is to be positioned adjacent the anal verge.

The open end of the inner body is connected in sealed relation to the outer expandable cover of the dilator and is adapted to be fitted with a valve assembly for the introduction of a fluid source therein. Accordingly, fluid entering the inner body of the dilator is forced through the perforations in its sidewalls to the outer expandable cover. The outer cover of the dilator is formed of a relatively flexible medical grade material, at least certain portions of which are suitably flexible to expand outwardly in response to pressure generated by the introduction of an expansion fluid within the dilator. Thus, when the dilator is inserted into the anal canal, it is designed to expand progressively in segments. Expansion may be gradually increased by the user as directed, and then decreased to its original dimensions, with a purpose of conditioning the anus for insertion of desired objects.

In use, the elongated tubular portions of the dilator are designed to be inserted fully into the anal canal. The distal segment which will be positioned within the rectum, is expanded first. The action of the distal segment expansion exerts pressure against the rectal wall resulting in a recto anal inhibitory reflex (RAIR) which causes a natural and involuntary relaxation of the internal anal sphincter muscles. Further expansion of the apparatus from the rectal segment to the proximal segment, positioned within the anal canal, stretches the canal from the anal verge to the rectum. By repeating the expansion process to achieve larger dimensional apparatus expansion, further stretching and relaxation of surrounding muscles and tissue will occur. The apparatus may be used prior to intercourse as well as repeatedly to train the anorectal canal to either normalize to a larger internal dimension, or to increase flexibility and distensibility. Such training activity may also condition the anorectal tissues to accommodate insertions with lower risk of physical tissue damage.

Methods set out in the claims and clauses that follow may be intended to provide a method of stretching and relaxing the muscles surrounding the anus for recreational purposes only. It may be that the methods do not include methods of stretching and relaxing the muscles surrounding the anus and anal canal that provide any sort of therapeutic or prophylactic effect.

The foregoing and additional features and advantages of the present invention will be more readily apparent from the following detailed description. It should be understood, however, that the description and specific examples herein are intended for purposes of illustration only and are not intended to limit the scope of the present disclosure.

### Brief Description of the Drawings

The drawings described herein are for illustration purposes only and are not intended to limit the scope of the present disclosure in any way.
Fig. 1 is a perspective view of recto-anal sequenced expander assembly which incorporates the principles of our invention;
Fig. 2 is a perspective view of the recto-anal sequenced expander assembly shown in Fig. 1 with a hose nipple attachment for attaching a source of fluid;
Fig. 3 is an exploded view of the recto-anal sequenced expander assembly shown in Fig. 1 with the hose nipple attachment therefor;
Fig. 4A is a diagrammatic side elevation view of a recto-anal sequenced expander assembly constructed in accordance with Fig. 3, showing the apparatus in its pre-expanded state;
Fig. 4B is a diagrammatic side elevation view of the recto-anal sequenced expander of Fig. 4A, showing the apparatus in a partially expanded state;
Fig. 4C is a diagrammatic side elevation view of the recto-anal sequenced expander of Fig. 4A, showing the apparatus in its fully expanded state;
Fig. 5 a diagrammatic side elevation view of the recto-anal sequenced expander of Fig. 4A, showing one possible configuration and dimensions of the device;
Fig. 6A is a vertical cross-sectional view of an alternative scissor-like mechanical expansion mechanism, showing the device in its unexpanded state;
6B is a vertical cross-sectional view of the scissor-like mechanical expansion mechanism shown in Fig. 6A, showing the device in its partially expanded state;
6C is a vertical cross-sectional view of the scissor-like mechanical expansion mechanism shown in Fig. 6A, showing the device in its fully expanded state;

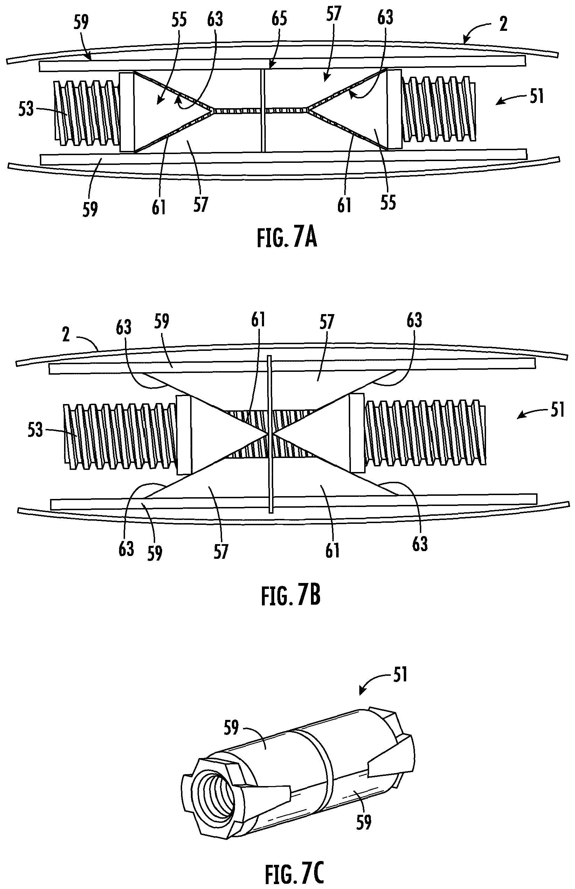

Fig. 7A is a vertical cross-sectional view of an alternative screw-type mechanical expansion mechanism, showing the device in its unexpanded state;
Fig. 7B is a vertical cross-sectional view of the screw-type mechanical expansion mechanism shown in Fig. 7A, showing the device in its fully expanded state; and
Fig. 7C is a perspective view of a screw-type mechanical expansion mechanism similar to that shown in Figs. 7A and 7B, showing the arched or curved expansion plates thereof.

### Detailed Description

The following description is merely exemplary in nature and is not intended to limit the present disclosure, application, or uses. It should be understood that throughout the drawings, corresponding reference numerals indicate like or corresponding parts and features.

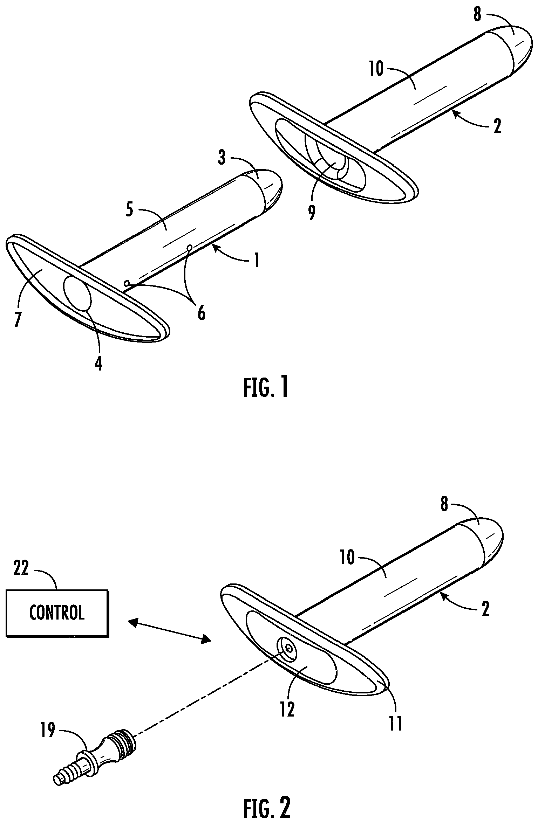

With reference now to Fig. 1 of the drawings, it is seen that one embodiment of the recto-anal expander or dilator assembly of the present invention is comprised generally of a relatively rigid inner tubular body member 1 which is insertable within an outer relatively flexible and expandable cover or sheath 2. The inner tubular body 1 of the dilator is closed at its distal end 3 and open at its proximal end 4 to allow for the introduction of an expansion fluid (not shown) therewithin. The cylindrical sidewall 5 of the inner body member 1 extending between opposite ends 3 and 4 thereof is perforated to provide openings 6 which allow for passage of the expansion fluid from the inner tubular body 1 to the outer expandable cover or sheath 2. Surrounding the open end 4 of the tubular body 1 and protruding outwardly therefrom in opposite directions is a handle member 7 which may be used for manual manipulation of the inner tube 1 during use.

The outer expandable cover or sheath 2 also has a tubular configuration including a closed distal end 8, an opposing open end 9, and a flexible cylindrical sidewall section 10 extending therebetween. As shown in Figs. 1 and 2, the cover 2 of the dilator assembly is compliantly configured and shaped to telescopically receive the inner body member 1 therewithin, which may be accomplished by insertion of the inner body distal end 3 into the open end 9 of the cover 2. Although the present embodiment discloses a single cover or sheath 2 covering the inner body member 1, it is conceivable that the cover 2 may take the form of multiple layers or sections without departing from the invention herein.

As shown in the drawings, a corresponding handle member 11 formed at the open end 9 of cover 2 is compliantly shaped to receive the handle member 7 of the inner body member 1. As best seen in Fig. 2, an annular seal 21 formed at the peripheral portions of the handle(s) 7 and 11 provide a fluid seal between the inner tubular body member 1 and outer cover 2 when assembled.

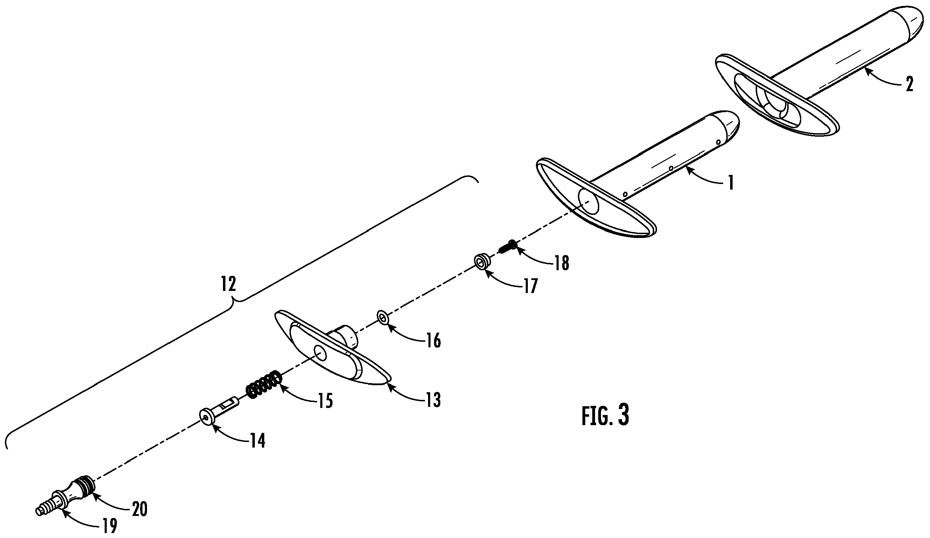

As further shown in Figs. 2 and 3, the handle member 7 and open end 4 of the inner tubular member 1 are adapted to be fitted in sealed relation with a valve assembly 12, which is similar in style to a conventional Schrader valve, well know the those skilled in the art of valves. As shown best in the exploded view of Fig. 3, the valve assembly 12 is comprised of a main valve body 13 which receives a valve stem 14 and spring15 in sealed relation within its central opening. An O-ring 16, valve cap 17 and screw 18 connect to the valve stem 14 and valve body 13 to complete the fluid valve assembly 12. As shown in Figs. 2 and 3, a valve nipple 19 is provided with O-ring 20 for connecting a fluid supply source (not shown) to the valve body 13 that feeds the inner body member 1 of the recto-anal expander.

It is contemplated that the outer cover or sheath 2 of the dilator shall be formed of a relatively flexible and expandable medical grade material, such as a relatively thin medical grade silicone. Of course, it is certainly conceivable that other flexible medical grade materials may also be available to serve the above purpose without departing from the invention herein. In a preferred embodiment, as will be explained further below, at least certain portions or segments of the sidewall 10 of the cover 2 may be constructed thinner or of a lower modulus of elasticity than others, or otherwise be capable of more readily expanding to a greater diameter than other portions of the cover 2. Thus, upon introduction of an expansion fluid into the body of cover 2, the expansion of various portions of the expandable cover 2 may progress sequentially along the length thereof.

The inner tubular body 1, on the other hand, shall be formed of a more rigid medical grade material, such as a semi-rigid medical grade silicone, which has sufficient structural integrity to support being inserted through the anus and into the anal canal. Of course, here again, it is certainly conceivable that other semi-rigid medical grade materials may also be available to serve the above purpose without departing from the invention herein. Accordingly, upon insertion of the recto-anal expander into one's anal canal, fluid entering the inner body 1 of the dilator will be forced through the perforations 6 in its sidewalls 5 of body 1 and into the outer expandable cover 2.

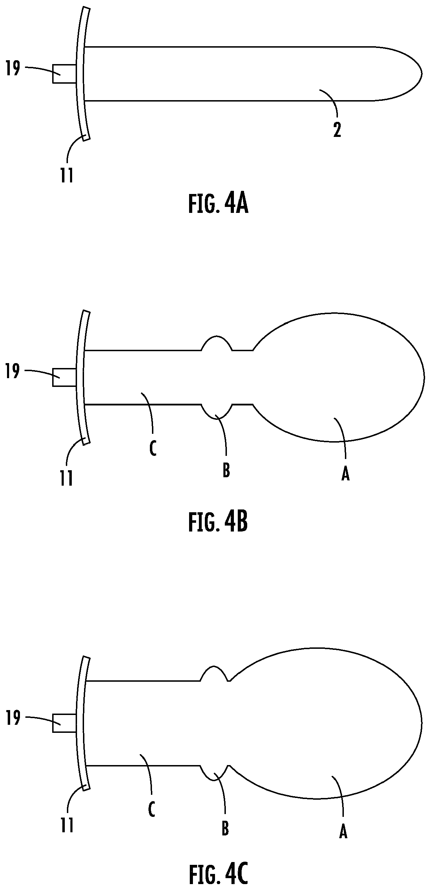

With reference now to Figs. 4A - 4C, the manner of use of the present invention will be described in more detail. In its assembled state (see, Fig. 2), the elongated tubular body portions (1, 2) of the expander are designed to be inserted fully into the anal canal. As seen in Figs. 4A, the recto-anal expander is shown in an unexpanded state, ready for insertion. In Fig. 4B, with the introduction of an expansion fluid, the recto-anal expander enters its first expansion stage where the rectal chamber section "A" and a sphincter ridge section "B" (discussed below) begin to expand. Finally, in Fig. 4C, the recto-anal expander becomes fully expanded, where the anal canal section "C" also expands. Notably, at this stage, the sphincter ridge "B" is still present, thus preventing inadvertent expulsion of the apparatus from the anus.

The sphincter ridge "B," as discussed above, is formed in a manner which maintains or creates a rim of greater diameter than the surrounding surface of the apparatus. As seen in Figs. 4B and 4C, this ridge "B" is formed at a location which positions the rim distally relative to the anal sphincter in order to maintain the position of the apparatus and aid in preventing inadvertent expulsion of the apparatus from the anus. Accordingly, the apparatus utilizes the normal anatomical sphincter muscle pressure, which is greater than the pressures exerted distally in the anorectal canal from the anal sphincter, to hold the apparatus in a desired position.

Therefore, the recto-anal expander of the present invention is designed to expand progressively in segments, beginning with distal end (Section "A") positioned in the rectal chamber, having a sphincter ridge (Section "B"), and finishing at or near the anal verge at the proximal end of the device (Section "C"). The distal segment which will be positioned within the rectum, is expanded first. The action of the distal segment expansion exerts pressure against the rectal wall, resulting in a recto anal inhibitory reflex (RAIR), which causes a natural and involuntary relaxation of the internal anal sphincter muscles. Further expansion of the apparatus from the distal rectal segment to the proximal segment, positioned within the anal canal, stretches the canal from the anal verge to the rectum.

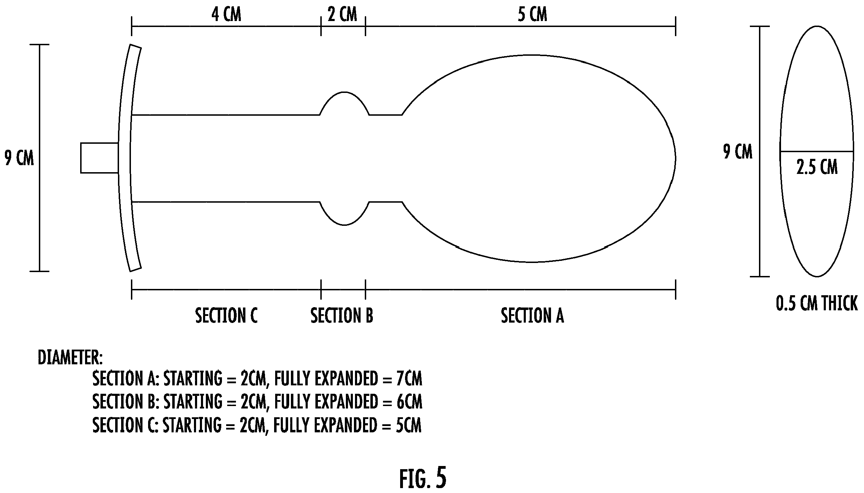

By repeating the expansion process to achieve larger dimensional apparatus expansion, further stretching and relaxation of surrounding muscles and tissue will occur. The apparatus may be used prior to intercourse as well as repeatedly to train the anorectal canal to either normalize to a larger internal dimension, or to increase flexibility and distensibility. Such training activity may also condition the anorectal tissues to accommodate insertions with lower risk of physical tissue damage. With reference to Fig. 5, one possible configuration of a recto-anal sequenced expander constructed in accordance with the present invention is shown, with possible dimensions contemplated for the expanded and unexpanded states of the device.

Various alternative embodiments and associated methods for sequentially expanding and reducing the diameter of the recto-anal expander are also contemplated for the purpose of distending the anorectal canal. In one preferred embodiment, as discussed above, it is contemplated to introduce a source of fluid to cause the desired expansion of the device, i.e., the apparatus may be expanded by increasing and decreasing a volume of air or liquid.

In some embodiments, it is contemplated that the fluid volume in the apparatus may be increased and decreased by manually applying pressure to an external or internal bladder, bulb, syringe, or other device which can hold and expel or withdraw a volume of fluid. In some embodiments, the volume may be increased and decreased using an external control device 22, such as one or more motors or pumps. In this regard, actuation may be directed by hydraulic or pneumatic pistons.

In some embodiments, the apparatus may be expanded by sequentially increasing the volume of balloons or bladders. Sequential filling, such that the distal balloon will expand before one or more additional balloons proximal to the first balloon, may be achieved using various valve-type systems. Such sequential filling may also be achieved using balloons or bladders of different physical expansion properties or elastic moduli, similar to that discussed above. Still further, such sequential filling could be achieved by the use of a manifold system which couples and decouples flow pathways.

In still other embodiments, it is contemplated that the apparatus may be expanded by mechanically causing plates to move outward against the surface of the apparatus. Still further, actuation of the device may be directed by a screw turning gears of various ratios. In other embodiments, this actuation may be directed by extending and retracting scissor type mechanisms. In some embodiments, mechanical actuation may be performed by motorized control means.

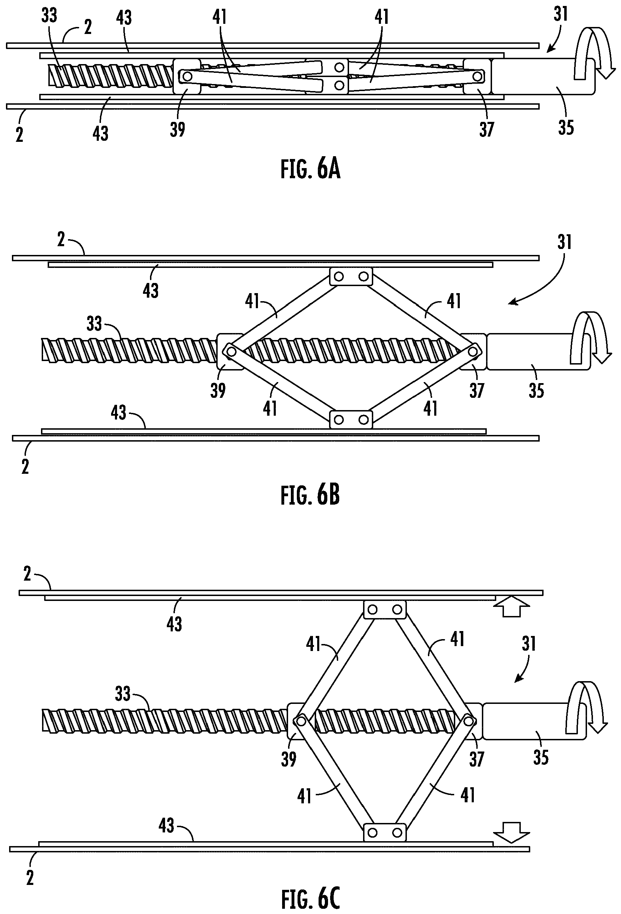

Examples of these types of mechanical expansion mechanisms can be seen in Figs. 6A - 6C, and in Figs. 7A - 7C. In Figs. 6A - 6C, a scissor type expansion mechanism 31 is shown which is driven via a threaded rod 33 connected to either a manual or motorized actuator 35. In the embodiment shown, the scissor-like expander 31 includes a stationary coupler 37 positioned adjacent the actuator 35 and a threaded expansion slide 39 movably carried by the threaded rod 33. Linkage arms 41 interconnect the stationary coupler 37 and movable slide 39 to outer expansion plates 43. The expansion mechanism 31 may be incorporated as a part of the insertable member 1 of the dilator, such that expansion thereof will cause expansion of the outer flexible sheath 2.

Rotation of actuator 35 in either direction will cause axial movement of slide 39 in one direction or the other along rod 33, thus effecting expansion or contraction of the plates 43 via linkage 41 in a predetermined area of the flexible sheath 2. As shown in Fig. 6A, in its unexpanded state, the scissor-type expansion mechanism 31 is elongated and relatively thin in diameter. As shown in Fig. 6B, upon rotation of actuator 35 in one direction, the threaded rod 33 will rotate and cause expansion slide 39 to move axially along rod 33. This, in turn, pushes linkage arms 41 outwardly, causing the expansion plates 43 to expand outwardly against the outer flexible sheath 2. As shown in Fig. 6C, continuing rotation of actuator 35 will ultimately cause the expansion mechanism to become fully expanded, where the actuator may be reversed to begin a contraction cycle in the localized area of sheath 2. This motion may be repeated, thus massaging, relaxing, and preconditioning of the muscles within the anal cavity.

In an alternative embodiment, it is contemplated that both couplers 37 and 39 could be threadedly connected to rod 33, albeit in opposite directions (right-handed vs. left-handed). In this case, the linkage arms 41 will cause expansion and contraction of plate 43 at any desired location along the length of rod 33. In this manner, one can control or establish predetermined areas along the length of rod 33, and thus along the expandable dilator, where expansion and contraction will take place.

In keeping with the above, it is also contemplated that one or more expansion mechanisms 31 may be incorporated as part of the insertable body member 1. Such expansions mechanisms 31 may be positioned within the outer sheath 2 of the recto-anal dilator at different locations to cause the desired sequential and progressive expansion of the dilator walls. Multiple expansion mechanisms 31 may be positioned along the length of the body member 1 in ninety-degree (90°) orientation relative to one another, thus providing expansion around the full circumference of the dilator. Also, the expansion plates may be formed with an arch shaped cross section to define a general cylindrical shape conforming to the insertable body 1 and outer sheath 2, thus helping to provide consistent radial expansion of the sheath 2 around the full circumference of the dilator.

Still further, it is also contemplated that the thread pitch of the slide members (37, 39) for different expansion mechanisms 31 may vary from one to another to establish different rates of expansion/contraction at different locations along the length of the dilator. In this manner, expansion and contraction of the outer sheath 2 may be progressively and sequentially controlled between the distal and proximal ends of the dilator.

In Figs. 7A - 7C, an alternative screw-type expansion mechanism 51 is shown that may be incorporated into the insertable body 1 of the dilator. In this embodiment, expansion mechanism 51 includes a threaded rod 53 rotationally connected to a manual or motorized actuator (not shown). Rod 53 carries a pair of oppositely facing tapered expansion slides 55 which function to movably engage a pair of expanders 57. In turn, the expanders 57 are connected to opposing expansion plates 59 which engage the outer flexible sheath 2 of the dilator.

As shown best in Figs. 7A and 7B, the expansion slides 55 are generally wedge or cone-shaped with oppositely facing outer tapered surfaces 61 which are adapted to mate with the tapered surfaces 63 of expanders 57. The opposing expansion slides 55 are threaded oppositely relative to one another, such that each slide travels in an opposite direction along rod 53 upon rotation thereof in either direction. A tension spring 65 connects the expanders 57 together such that they are drawn together naturally when the dilator is in its unexpanded state.

As shown in Fig. 7A, the expansion slides 55 are positioned apart from one another when the dilator is in its unexpanded state. In this case, the body member 1 is thinner in diameter to facilitates penetration through the anus. In Fig. 7B, the dilator body is shown in an expanded state, where rotation of rod 53 has caused the expansion slides 55 to travel toward one another, thus pushing the sliding expanders 57 radially outward against the opposing expansion plates 59. Accordingly, the outer sheath 2 in the area of the expansion mechanism 51 is caused to expand.

Like the expansion mechanism 31 in embodiment of Figs. 6A - 6C, it is contemplated that multiple expansion mechanisms 51 may be incorporated as part of the insertable body member 1. Such expansion mechanisms 51 may be incorporated into the insertable body member 1 and positioned within the outer sheath 2 of the recto-anal dilator at different locations to cause the desired sequential and progressive expansion of the dilator walls. The wedge or cone shape of the expansion slides 55 will function to provide an even outward radial pressure against the expanders 57 and consequently against the expansion plates 59. Here again, as seen best in Fig. 7C, the expansion plates 59 may be formed with an arch shaped cross section to define a general arcuate or cylindrical shape, thereby providing consistent radial expansion of the sheath 2 around the full circumference of the dilator.

With the expansion mechanism 51, the thread pitch of the expansion slides 55 for different expansion mechanisms 51 may also vary from one to another to establish different rates of expansion/contraction at different locations along the length of the dilator. Here again, expansion and contraction of the outer sheath 2 may be progressively and sequentially controlled between the distal and proximal ends of the dilator by adjustment of the rate of expansion of the separate expansion mechanisms 51.

In still further embodiments, it is contemplated that an external expansion mechanism may be disconnected from the apparatus while the apparatus maintains the set expansion dimension. This may allow the user to maintain the anorectal position of the apparatus for a period of time and to move freely without the external mechanism affixed.

It is also contemplated that the expansion and reduction of the apparatus may be measured, quantified, and/or scaled, and the information thereof may be displayed, on the apparatus, on an extension of the apparatus, and/or on a remote device. In some embodiments, measuring, quantifying, and/or scaling may be performed using mechanical means, using visual indication, and/or using one or more sensors. In some embodiments, the display of measuring, quantifying, and/or scaling may also use alpha or numeric indicators, color-coded indicators, graphical indicators, and/or words.

Still further, it is contemplated that the measuring, quantifying, and/or scaling of the expansion and reduction of the apparatus may be recorded by mechanical, analog, or digital methods. In some embodiments, such recorded information may be transferred by mechanical, analog, or digital methods to another device or media.

The method of expansion and reduction of the apparatus may also be directed by a timing mechanism. This timing mechanism may be used to regulate the rate of expansion and reduction of the apparatus. In some embodiments, the timing mechanism may be integrated within the apparatus. In other embodiments, the timing mechanism may be integrated within an ancillary control device 22, such as a mobile phone or remote controller, and/or a software application. In some embodiments, the timing may be displayed by use of alpha or numeric indicators, color-coded indicators, graphical indicators, and/or words. In some embodiments, the timing may be recorded by mechanical, analog, or digital methods. Such recorded information may be transferred by mechanical, analog, or digital methods to another device or media.

Methods and apparatus involving Bluetooth, WIFI, infrared, or another wireless technologies may also be employed to communicate information and/or functional or non-functional instructions between the apparatus and an ancillary control device 22 such as a mobile phone or remote controller, and/or software application. Such functionality could be used to direct the operation of the apparatus, such as to expand or reduce the dimensions of the apparatus, or to incorporate mechanical, acoustic, or other devices to cause the apparatus to induce vibrations of determined frequencies. Controlled operation of pressure transducers may also be integrated to measure pressures exerted upon the apparatus by the recto anal musculature.

It is also contemplated that a controlling device 22 could be connected to the apparatus using wires, pneumatic tubes, hydraulic tubes, or mechanical methods, to direct the operation of the apparatus such as to expand or reduce the dimensions of the apparatus.

Finally, in still further embodiments, a disposable protective and/or lubricious sheath, possibly in the manner of a condom, may be utilized to cover the apparatus during insertion into the anorectal canal. Such a sheath would aid cleaning and maintenance of the apparatus. In some embodiments, the apparatus may include mechanisms or methods to affix the sheath to the apparatus to aid in preventing undesired movement or inadvertent displacement of the sheath from the apparatus.

Certain terminology is used herein for purposes of reference only, and thus is not intended to be limiting. For example, terms such as "upper", "lower", "above", "below", "top", "bottom", "upward", "downward", "rearward", and "forward" may refer to directions in the drawings to which reference is made. Terms such as "distal", "proximal", "front", "back", "rear", "bottom" and "side", may describe the orientation of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second" and other such numerical terms referring to structures do not imply a sequence or order unless clearly indicated by the context.

When introducing elements or features and the exemplary embodiments, the articles "a", "an", "the" and "said" are intended to mean that there are one or more of such elements or features. The terms "comprising", "including" and "having" are intended to be inclusive and mean that there may be additional elements or features other than those specifically noted. It is further to be understood that the method steps, processes, and operations described herein are not to be construed as necessarily requiring their performance in the particular order discussed or illustrated, unless specifically identified as an order of performance. It is also to be understood that additional or alternative steps may be employed.

The disclosure herein is intended to be merely exemplary in nature and, thus, variations that do not depart from the gist of the disclosure are intended to be within the scope of the disclosure. Such variations are not to be regarded as a departure from the spirit and scope of the disclosure which comprises the matter shown and described herein, and set forth in the appended claims.

The following claim-like clauses also form part of the disclosure relating to the present invention:

### Claim-like clauses

[Clause 1] A recto-anal sequenced expander for relaxing muscles surrounding the anus and anal canal, comprising:
(a) an elongated anal insertion member having a distal end portion for penetrating the anus and an opposing proximal end portion, at least said distal end portion of said insertion member having a wall structure of sufficient rigidity to permit penetration through the anus to the anal canal; and
(b) said insertion member having an expansion mechanism incorporated therein which operatively expands said insertion member radially outward in a sequential and progressive manner from an area adjacent said distal end toward an area adjacent said proximal end of said insertion member.

[Clause 2] The recto-anal sequenced expander set forth in Clause 1, wherein said insertion member is comprised of an elongated tubular member having a closed distal end, an open proximal end, and a perforated sidewall structure extending therebetween which is enclosed within an outer flexible sheath.

[Clause 3] The recto-anal sequenced expander set forth in Clause 2, wherein said expansion mechanism is comprised of a fluid and said open proximal end of said tubular member provides an input port for the introduction of said fluid into said tubular member and through said perforated sidewall structure to said outer sheath.

[Clause 4] The recto-anal sequenced expander set forth in Clause 2, wherein said outer sheath is constructed of alternating sidewall sections having varying degrees of material flexibility.

[Clause 5] The recto-anal sequenced expander set forth in Clause 1, wherein said expansion mechanism is a member selected from the group consisting of a gaseous substance, a liquid substance, a semi-liquid substance, and a mechanical actuator.

[Clause 6] The recto-anal sequenced expander set forth in Clause 2, wherein said expansion mechanism and said outer sheath cooperatively interact to form a circumferentially expanded rim section intermediately located between said proximal end portion and said distal end portion of said insertion member which helps to prevent inadvertent expulsion of the expander from the anal canal.

[Clause 7] The recto-anal sequenced expander set forth in Clause 6, wherein said expansion mechanism interacts with said outer sheath to cause an initial expansion of said distal end portion of said sheath and said circumferential rim section thereof, followed be a progressive expansion of said sheath therebetween from said distal end portion toward said rim.

[Clause 8] The recto-anal sequenced expander set forth in Clause 1, wherein said insertion member includes an outer surrounding flexible sheath which is affixed in sealed relation to said proximal end portion of said insertion member, and said proximal end portion thereof is constructed to facilitate the introduction of an expansion fluid into said insertion member for passage to said outer sheath through a plurality of openings extending through a sidewall of said insertion member.

[Clause 9] The recto-anal sequenced expander set forth in Clause 8, wherein said insertion member includes a first handle member formed adjacent said proximal end portion which protrudes outwardly therefrom, and said outer sheath includes a second handle member cooperatively shaped to mate with said first handle member in sealed relation thereto.

[Clause 10] The recto-anal sequenced expander set forth in Clause 8, including a control device for effecting at least one of a group consisting of (i) measuring the expansion of said outer sheath; (ii) displaying the expansion of said outer sheath; (iii) recording the expansion of said outer sheath; (iv) controlling expansion and reversion of said outer sheath; (v) cycling expansion and reversion of said outer sheath over time; (vi) incorporating an integrated timer for controlling expansion and reversion of said outer sheath; (vii) measuring the amount of pressure exerted upon said outer sheath; (viii) inducing vibration to the expander; and (ix) remotely controlling the expander through wireless communication.

[Clause 11] The recto-anal sequenced expander set forth in Clause 1, wherein said insertion member is comprised of an elongated tubular member with an input port for the introduction of an expansion fluid and perforations therein extending to an outer surrounding flexible sheath, said outer sheath being constructed with sections of varying material flexibility to facilitate sequential progressive expansion thereof by said expansion fluid.

[Clause 12] A recto-anal sequenced expander for relaxing muscles surrounding the anus and anal canal, comprising:
(a) a tubular insertion member with a closed distal end, an open proximal end, and a perforated sidewall structure extending therebetween, said distal end being tapered to permit penetration through the anus and into the anal canal;
(b) an outer flexible sheath surrounding a substantial portion of said insertion member between said distal end and said proximal end thereof, said sheath having an imperforate wall structure and being sealed around said perforated sidewall structure of said insertion member;
(c) an input valve connected to said proximal end of said insertion member for connection to a fluid supply source; and
(d) said wall structure of said outer sheath being constructed to balloon outwardly around said insertion member progressively from said distal end of said insertion member toward said proximal end upon the introduction of a fluid from said fluid supply source through said input valve.

[Clause 13] The recto-anal sequenced expander set forth in Clause 12, wherein said wall structure of said outer sheath has multiple wall sections of varying degrees of material flexibility to facilitate sequential expansion of said wall sections progressively from said distal end of said insertion member toward said proximal end.

[Clause 14] The recto-anal sequenced expander set forth in Clause 12, wherein said wall structure of said outer sheath has multiple wall sections of varying degrees of material flexibility extending between said distal end and said proximal end of said insertion member which facilitate expansion of said wall sections at different rates.

[Clause 15] The recto-anal sequenced expander set forth in Clause 14, wherein one of said wall sections of said sheath located between said distal end and said proximal end of said insert member is formed of a material having a lower elastic modulus than adjacent said wall sections of said sheath, thus permitting greater and more rapid expansion thereof to prevent against inadvertent expulsion of the expander from the anus.

[Clause 16] The recto-anal sequenced expander set forth in Clause 12, wherein said wall structure of said outer sheath has a first annular wall section located adjacent said distal end of said insert member which is adapted to expand progressively toward said proximal end of said insert member upon the introduction of said fluid therein, and a second annular wall section spaced proximally from said first wall section, said second wall section being constructed to expand more rapidly than adjacent wall sections of said outer sheath thereto, thus forming a circumferential rim to bear against the anal sphincter and prevent inadvertent expulsion of the expander from the anus.

[Clause 17] The recto-anal sequenced expander set forth in Clause 12, including a control device for effecting at least one of a group consisting of (i) measuring the expansion of said outer sheath; (ii) displaying the expansion of said outer sheath; (iii) recording the expansion of said outer sheath; (iv) controlling expansion and reversion of said outer sheath; (v) cycling expansion and reversion of said outer sheath over time; (vi) incorporating an integrated timer for controlling expansion and reversion of said outer sheath; (vii) measuring the amount of pressure exerted upon said outer sheath; (viii) inducing vibration to the expander; and (ix) remotely controlling the expander through wireless communication.

[Clause 18] A method of stretching and relaxing the muscles surrounding the anus and anal canal, comprising the steps of:
(a) providing an elongated anal insertion member with a distal end portion and an opposing proximal end portion, said insertion member including an expansion mechanism incorporated therein for causing radial expansion of said insertion member sequentially and progressively from said distal end portion toward said proximal end portion;
(b) inserting said anal insertion member distal end first through the anus and into the anal canal; and
(c) activating said expansion mechanism of said anal insertion member to cause gradual sequential and progressive distension of the muscles surrounding the anus and anal canal from said distal end portion of said insertion member toward said proximal end portion thereof.

[Clause 19] The method of stretching and relaxing the muscles surrounding the anus and anal canal set forth in Claim 18, wherein said step of activating said expansion mechanism includes introducing a fluid within the insertion member.

[Clause 20] The method of stretching and relaxing the muscles surrounding the anus and anal canal set forth in Claim 19, including the step of providing said anal insertion member with an inner tubular member having a perforated sidewall and an outer flexible sheath which surrounds said perforated sidewall, wherein said inner tubular member includes a port at said proximal end portion of said insertion member which facilitates the introduction of said fluid.

[Clause 21] The method of stretching and relaxing the muscles surrounding the anus and anal canal set forth in Claim 18, including repeating step (c) to help relax and condition the muscles surrounding the anus and anal canal.

## Claims

1. A recto-anal sequenced expander for relaxing muscles surrounding the anus and anal canal, comprising:
(b) an elongated anal insertion member (1, 2) having a distal end portion (3, 8) for penetrating the anus and an opposing proximal end portion (4, 9), at least said distal end portion (3, 8) of said insertion member (1, 2) having a wall structure (5, 10) of sufficient rigidity to permit penetration through the anus to the anal canal;
**characterized in that**
said insertion member (1, 2) having an expansion mechanism (12, 31, 51) incorporated therein which operatively expands said insertion member (1, 2) radially outward in a sequential and progressive manner from an area adjacent said distal end portion (3, 8) toward an area adjacent said proximal end portion (4, 9) of said insertion member (1, 2).

2. The recto-anal sequenced expander set forth in Claim 1, wherein said insertion member (1, 2) is comprised of an elongated tubular member (1) having a closed distal end (3), an open proximal end (4), and a perforated sidewall structure (5) extending therebetween which is enclosed within an outer flexible sheath (2).

3. The recto-anal sequenced expander set forth in Claim 2, wherein said expansion mechanism (12) is comprised of a fluid and said open proximal end (4) of said tubular member (1) provides an input port (4) for the introduction of said fluid into said tubular member (1) and through said perforated sidewall structure (5) to said outer sheath (2).

4. The recto-anal sequenced expander set forth in Claim 2, wherein said outer sheath (2) is constructed of alternating sidewall sections (A, B, C) having varying degrees of material flexibility.

5. The recto-anal sequenced expander set forth in Claim 1, wherein said expansion mechanism (12, 31, 51) is a member selected from the group consisting of a gaseous substance, a liquid substance, a semi-liquid substance, and a mechanical actuator.

6. The recto-anal sequenced expander set forth in Claim 2, wherein said expansion mechanism (12, 31, 51) and said outer sheath (2) cooperatively interact to form a circumferentially expanded rim section (B) intermediately located between said proximal end portion (4, 9) and said distal end portion (3, 8) of said insertion member (1, 2) which helps to prevent inadvertent expulsion of the expander from the anal canal.

7. The recto-anal sequenced expander set forth in Claim 6, wherein said expansion mechanism (12, 31, 51) interacts with said outer sheath (2) to cause an initial expansion of a distal end section (A) of said sheath (2) and said circumferential rim section (B) thereof, followed by a progressive expansion of said sheath (2) therebetween from said distal end section (A) toward said rim section (B).

8. The recto-anal sequenced expander set forth in Claim 1, wherein said insertion member (1, 2) includes an outer surrounding flexible sheath (2) which is affixed in sealed relation to said proximal end portion (4, 9) of said insertion member (1, 2), and said proximal end portion (4, 9) thereof is constructed to facilitate the introduction of an expansion fluid into said insertion member (1, 2) for passage to said outer sheath (2) through a plurality of openings (6) extending through a sidewall (5) of said insertion member (1, 2).

9. The recto-anal sequenced expander set forth in Claim 8, wherein said insertion member (1, 2) includes a first handle member (7) formed adjacent said proximal end portion (4) which protrudes outwardly therefrom, and said outer sheath (2) includes a second handle member (11) cooperatively shaped to mate with said first handle member (7) in sealed relation thereto.

10. The recto-anal sequenced expander set forth in Claim 8, including a control device (22) for effecting at least one of a group consisting of (i) measuring the expansion of said outer sheath (2); (ii) displaying the expansion of said outer sheath (2); (iii) recording the expansion of said outer sheath (2); (iv) controlling expansion and reversion of said outer sheath (2); (v) cycling expansion and reversion of said outer sheath (2) over time; (vi) incorporating an integrated timer for controlling expansion and reversion of said outer sheath (2); (vii) measuring the amount of pressure exerted upon said outer sheath (2); (viii) inducing vibration to the expander; and (ix) remotely controlling the expander through wireless communication.

11. The recto-anal sequenced expander set forth in Claim 1, wherein said insertion member (1, 2) is comprised of an elongated tubular member (1) with an input port (4) for the introduction of an expansion fluid and perforations (6) therein extending to an outer surrounding flexible sheath (2), said outer sheath (2) being constructed with sections (A, B, C) of varying material flexibility to facilitate sequential progressive expansion thereof by said expansion fluid.

12. A method of stretching and relaxing the muscles surrounding the anus and anal canal, comprising the steps of:
(d) providing an elongated anal insertion member (1, 2) with a distal end portion (3, 8) and an opposing proximal end portion (4, 9), said insertion member (1, 2) including an expansion mechanism (12, 31, 51) incorporated therein for causing radial expansion of said insertion member (1, 2) sequentially and progressively from said distal end portion (3, 8) toward said proximal end portion (4, 9);
(e) inserting said anal insertion member (1, 2) distal end first through the anus and into the anal canal; and
(f) activating said expansion mechanism (12, 31, 51) of said anal insertion member (1, 2) to cause gradual sequential and progressive distension of the muscles surrounding the anus and anal canal from said distal end portion (3, 8) of said insertion member (1, 2) toward said proximal end portion (4, 9) thereof.

13. The method of stretching and relaxing the muscles surrounding the anus and anal canal set forth in Claim 12, wherein said step of activating said expansion mechanism (12) includes introducing a fluid within the insertion member (1, 2).

14. The method of stretching and relaxing the muscles surrounding the anus and anal canal set forth in Claim 13, including the step of providing said anal insertion member (1, 2) with an inner tubular member (1) having a perforated sidewall (5) and an outer flexible sheath (2) which surrounds said perforated sidewall (5), wherein said inner tubular member (1) includes a port (4) at said proximal end portion (4) of said insertion member (1, 2) which facilitates the introduction of said fluid.

15. The method of stretching and relaxing the muscles surrounding the anus and anal canal set forth in Claim 12, including repeating step (c) to help relax and condition the muscles surrounding the anus and anal canal.
